# EUROPÄISCHE PATENTSCHRIFT

(11) **EP 3 749 254 B1**
(45) Veröffentlichungstag und Bekanntmachung des Hinweises auf die Patenterteilung: **22.12.2021**
(21) Anmeldenummer: 19712124.7
(22) Anmeldetag: 05.02.2019
(51) Int. Cl.: A61F 2/24

(54) **VORRICHTUNG ZUR PRÜFUNG DER FUNKTION EINER AORTENKLAPPE**
DEVICE FOR TESTING THE FUNCTIONING OF AN AORTIC VALVE
DISPOSITIF POUR LE CONTRÔLE DU FONCTIONNEMENT D'UNE VALVE AORTIQUE

(30) Priorität: 09.02.2018 DE 102018102940
(43) Veröffentlichungstag der Anmeldung: 16.12.2020
(73) Patentinhaber: Carrero Gomez, Francisco Javier, 32545 Bad Oeynhausen (DE); Bogatzki, Michael, 32429 Minden (DE); SPANDAUVENTURES GMBH, 12205 Berlin (DE)
(72) Erfinder: CARRERO GOMEZ, Francisco Javier, 32545 Bad Oeynhausen (DE)
(74) Vertreter: Hamm&Wittkopp Patentanwälte PartmbB
(86) Internationale Anmeldenummer: PCT/DE2019/100120
(87) Internationale Veröffentlichungsnummer: WO 2019/154461

(56) Entgegenhaltungen:
- WO-A1-2014/000105
- WO-A2-2012/049679
- US-A- 5 489 298
- US-A1- 2011 071 351
- US-A1- 2013 297 009
- US-A1- 2015 359 633
- US-B1- 6 491 624

## Beschreibung

### Technisches Umfeld

Die Erfindung betrifft eine Vorrichtung zur Prüfung der Funktion einer Aortenklappe durch Aufsetzen der Vorrichtung auf das freie Ende einer zylinderförmigen in die Aorta eingesetzten Rohrprothese, die mit ihrem anderen Ende an die Aortenwand im Bereich der Aortenklappe angebunden ist.

### Stand der Technik

14. A data processing method comprising:Eine Vorrichtung der eingangs beschriebenen Art ist nach Kenntnis des Anmelders bislang aus dem Stand der Technik im medizinischen Bereich nicht bekannt.

Üblich ist bislang bei Operationen im Bereich von Aortenklappen, deren Funktion beeinträchtigt ist, die in entsprechender Durchmessergröße an den Aderdurchmesser des Patienten angepasst eingesetzten Rohrprothesen mit der Aderwandung zu vernähen und die entsprechenden Anbindungen zum Herzkreislauf zu schaffen.

Sind im Rahmen der Operation alle Vernähungen vorgenommen, so werden die geschaffenen Verbindungen zwischen Rohrprothese und Körpergewebe durch Inaugenscheinnahme auf Dichtigkeit untersucht.

Da Operationen im Aortenklappenbereich kompliziert sind, beanspruchen sie in der Regel eine nicht unbeträchtliche Zeitspanne von bis zu drei Stunden. Sollte sich im abschließenden Zeitfenster der Operation herausstellen, dass in Bezug auf die Vernähung Nacharbeiten notwendig sind, so erfordert dies einen zusätzlichen Zeitaufwand. Statistisch gesehen erhöht sich das Komplikationsrisiko für den Patienten ab einer bestimmten Zeitspanne mit erhöhter Proportionalität, so dass der übliche Anteil an Komplikationen von ca. 1 % bei derartigen Eingriffen bei notwendigen ergänzenden Nacharbeiten auf in der Regel 3 bis 4 % steigt. US2011071351 A1 offenbart eine Vorrichtung zum Bewerten einer Aortenklappe nach dem Öffnen einer Aorta distal zu der Aortenklappe, die einen Körper umfasst, der eine Kammer definiert. Der Körper weist ein im Allgemeinen kreisförmiges proximales offenes Ende und ein distales Ende auf, wobei das proximale offene Ende konfiguriert ist, um reversibel an einer von der Aorta oder einem kurzen offenen Schlauch distal zu der Aortenklappe in einer im Allgemeinen wasserdichten Weise anbringbar zu sein. Zumindest ein Teil des Körpers ist transparent, um die Aortenklappe zu sehen.

### Aufgabe der Erfindung

Da es das Ziel aller operativen medizinischen Weiterentwicklung ist, den Prozentsatz an Komplikationen, denen ein Patient im Rahmen einer Operation ausgesetzt ist und die gegebenenfalls zum Tode des Patienten führen können, herabzusetzen, ist es Aufgabe der vorliegenden Erfindung, eine Vorrichtung der eingangs beschriebenen Art zu schaffen, mit der auf einfache Art und Weise die Funktion der Aortenklappe sowie der vorgenommenen Anbindung der verwendeten Rohrprothese unmittelbar geprüft werden kann, um die insgesamt notwendige Operationszeit herabzusetzen und das Patientenrisiko im Hinblick auf mögliche Komplikationen zu verringern.

### Lösung der Aufgabe

Die gestellte Aufgabe wird für eine Vorrichtung zur Prüfung der Funktion einer Aortenklappe mit den oben beschriebenen gattungsgemäßen Merkmalen durch die im kennzeichnenden Teil des Anspruches 1 angegebene Merkmalskombination gelöst.

Erfindungswesentlich dabei ist es, dass die Vorrichtung aus einem mehrteiligen, mit mindestens einem Durchflusskanal für Kontrollflüssigkeit versehenen Gehäuse besteht, wobei das Gehäuse an seiner dem freien Ende zugewandten Unterseite der Rohrprothese mit einer umlaufenden, auf den Rand der Rohrprothese aufsetzbaren Abdichtvorrichtung versehen ist und wobei an der Unterseite des Gehäuses mindestens ein Druckmesssensor und mindestens ein optisches Sensorelement zur Videoaufnahme vorhanden ist.

Die aufgeführte Merkmalskombination der erfindungsgemäßen Vorrichtung ermöglicht es, diese unmittelbar nach der Anbindung der Rohrprothese an das körpereigene Aortenwandmaterial des Patienten auf das gegenüber der Aortenklappe liegende freie Ende der Rohrprothese aufzusetzen und mittels der Abdichtvorrichtung luft- und flüssigkeitsdicht mit dem Randbereich der Rohrprothese zu verbinden.

Anschließend wird durch den im Gehäuse vorhandenen Durchflusskanal der unter der erfindungsgemäßen Vorrichtung vorhandene Raum der Rohrprothese oberhalb der Aortenklappe mit einer Kontrollflüssigkeit beaufschlagt, wobei durch die Kontrollflüssigkeit ein entsprechender Überdruck im Bereich der Aortenklappe aufgebaut werden kann. Durch den an der Vorrichtung vorhandenen Druckmesssensor ist eine Kontrolle des aufgebauten Druckes problemlos möglich.

Das zusätzlich vorhandene optische Sensorelement dient dazu, den Bereich oberhalb der Aortenklappe aufzunehmen und so eine visuelle Prüfung der Funktion der Aortenklappe vornehmen zu können.

Gleichzeitig können durch den Druckaufbau im Bereich vor der Aortenklappe dort hergestellte Anbindungen an den Herzkreislauf auf Dichtigkeit geprüft werden.

Der vorgesehene Einsatz der erfindungsgemäßen Vorrichtung ermöglicht erstmals eine Prüfung der durchgeführten Operationsmaßnahme, die unmittelbar Aufschluss über die Qualität des Operationsergebnisses geben kann. Das Risiko eines zusätzlichen Zeitaufwandes bei derartigen Operationen sinkt durch die Verwendung der erfindungsgemäßen Vorrichtung nicht unbeträchtlich, so dass insgesamt das Risiko von Komplikationen für den Patienten herabgesetzt wird.

Besondere Ausgestaltungen des Gegenstandes der Erfindung ergeben sich in Zusammenschau mit der technischen Lehre, wie sie im Anspruch 1 beschrieben ist zusätzlich aus den auf den Hauptanspruch rückbezogenen Unteransprüchen.

Hinsichtlich des Aufbaus der Vorrichtung hat es sich als vorteilhaft erwiesen, wenn der Durchflusskanal eine an der dem freien Ende der Rohrprothese abgewandten Oberseite angeordnete Eingangsöffnung aufweist und innerhalb des Gehäuses zu einem Durchflusskanal verzweigt ist, der an der Unterseite des Gehäuses innerhalb der umlaufenden Abdichtvorrichtung in eine ringförmige Auslassöffnung mündet. Diese Maßnahme führt zu einer leichten Zugänglichkeit des Durchflusskanals auf der einen Seite zur Beaufschlagung mit der Kontrollflüssigkeit und zum anderen zu einer gleichmäßigen und schnellen Befüllung des Bereiches der Rohrprothese zwischen Unterseite des Gehäuses und der Aortenklappe.

Zweckmäßig kann es darüber hinaus sein, den Aufbau des Gehäuses so zu gestalten, dass die Innenseite des Durchflusskanals durch ein zentrales Innenformteil als Bestandteil des Gehäuses gebildet ist, in den der Druckmesssensor und das optische Sensorelement aufgenommen sind, wobei die Messfläche und Aufnahmeöffnung an der Unterseite offenliegen. Dieses Innenformteil bildet ein zentrales Element der gesamten Vorrichtung und beinhaltet selbstverständlich die Verkabelung der elektronischen Elemente.

Die Außenseite des Durchflusskanals wird entsprechend einer zweckmäßigen Weiterbildung durch ein das zentrale Innenformteil beabstandet umgebendes glockenförmiges Außenformteil gebildet, in dem die Eingangsöffnung des Ringkanals angeordnet ist. Dieses Außenformteil kann gegebenenfalls vom Innenformteil getrennt werden und in unterschiedlicher Durchmesserdimension ausgestaltet sein.

Durch diese Maßnahme lässt sich eine Anpassung an unterschiedliche Durchmesser der Rohrprothese vornehmen, wobei die unterschiedlichen Durchmesser dem Querschnitt der Aorta angepasst sind, in der die Rohrprothese eingesetzt wird. Das Außenformteil wird aufgrund seiner Variantenmehrzahl vorzugsweise aus Kunststoff hergestellt.

Hinsichtlich des Aufbaus der erfindungsgemäßen Vorrichtung hat es sich darüber hinaus als vorteilhaft erwiesen, die Abdichtvorrichtung am Außenformteil abnehmbar durch eine Fixiervorrichtung festzulegen. Diese konstruktive Gestaltung ermöglicht es, je nach Anforderung gegebenenfalls unterschiedliche Varianten der Abdichtvorrichtung am Außenformteil anzubringen, und somit die Vielseitigkeit der erfindungsgemäßen Vorrichtung zu erhöhen.

Dabei sieht eine Variante der Abdichtvorrichtung vor, dass diese einen ringförmigen Gehäusekörper aufweist, der einen rechteckförmigen Querschnitt aufweist und bei dem an einer der gegenüberliegenden Schmalseiten des Querschnittes eine Ausnehmung zur Aufnahme eines Vorsprunges des Außenformteiles angeordnet ist und bei der sich an der gegenüberliegenden Schmalseite eine U-förmige Ausnehmung zur Aufnahme des ringförmigen oberen Endes der Rohrprothese befindet. Die Gestaltung mit jeweils U-förmigen Ausnehmungen erhöht die Festigkeit der jeweiligen Verbindung zwischen Außenformteil, Abdichtvorrichtung und Rohrprothese und dient gleichzeitig einer Verbesserung der Abdichtwirkung zwischen den in Rede stehenden Bauteilen.

Die Dichtwirkung zwischen Rohrprothese und Abdichtvorrichtung kann zusätzlich dadurch verbessert werden, dass der äußere Schenkel der U-förmigen Ausnehmung mindestens eine elastische Dichtmembran aufweist, die in Richtung des gegenüberliegenden Schenkels der U-förmigen Ausnehmung vorgespannt ist.

Die Vorspannung der Dichtmembran gewährleistet, dass der obere Rand der Rohrprothese innerhalb der U-förmigen Ausnehmung der Abdichtvorrichtung geklemmt wird und somit ein Ausfluss aus dem Innenraum der Rohrprothese bei aufgesetzter erfindungsgemäßer Vorrichtung und erhöhtem Druck der eingesetzten Kontrollflüssigkeit verhindert wird.

Eine weitere konstruktive Ausbildung der Abdichtvorrichtung sieht vor, dass die elastische Dichtmembran im Bereich der der Rohrprothese zugewandten U-förmigen Ausnehmung doppelwandig ausgeführt ist und sich zwischen den parallel beabstandeten Wänden der Dichtmembran ein Zwischenraum befindet, in den eine Mehrzahl von Federstäben über den Umfang des umlaufenden Zwischenraumes verteilt angeordnet sind, die die Vorspannung der Dichtmembran bewirken.

Durch diese konstruktive Gestaltung kann eine Aufgabentrennung der von der Dichtmembran zu leistenden Aufaaben herbeigeführt werden. Das Material der Dichtmembran kann bei dieser Ausgestaltung optimal auf die von ihr zu leistende Dichtwirkung im Hinblick auf die Oberflächenbeschaffenheit ausgerichtet werden, ohne besondere Elastizitätsanforderungen des Materials berücksichtigen zu müssen. Die verwendeten Federstäbe können dabei optimal für die aufzubringenden Federkräfte und somit für die Dichtwirkungskräfte angepasst werden.

Als zusätzlich vorteilhaft hat es sich bei dieser Gestaltung der Abdichtvorrichtung erwiesen, wenn die Federstäbe jeweils ein Knickgelenk aufweisen, wobei die Knickgelenke eine Winkellage des Teils der Dichtmembran in Richtung des Gehäuses oberhalb des Knickgelenkes von demjenigen Teil der Dichtmembran unterhalb des Knickgelenkes ermöglichen.

Die erfindungsgemäße Vorrichtung kann durch diese konstruktive Maßnahme besonders leicht auf das obere Ende der Rohrprothese aufgesetzt werden, da das untere Ende der Dichtmembran durch das Knickgelenk nach außen abgespreizt ist, so dass sich insgesamt ein vergrößerter Ringspalt zwischen innerem Schenkel der unteren U-förmigen Ausnehmung der Abdichtvorrichtung und dem äußeren Schenkel ergibt, was das Einführen des oberen Endes der Rohrprothese erleichtert. Ist die Vorrichtung auf das Ende der Rohrprothese aufgesetzt, so wird wiederum bedingt durch das Knickgelenk, der untere Teil der Dichtmembran an die Außenseite der Rohrprothese angeklappt und erzeugt insgesamt die für eine Abdichtung notwendigen Anpresskräfte.

Für spezielle Anwendungen kann es darüber hinaus vorteilhaft sein, wenn die zum gegenüberliegenden Schenkel der u-förmigen Ausnehmung zur Aufnahme der Rohrprothese weisende Innenfläche des Schenkels der Dichtmembran einen über die Innenfläche vorstehenden umlaufenden Druckwulst aufweist, der in aufgesetztem Zustand der Vorrichtung auf das freie Ende der Rohrprothese in eine korrespondierende Ausnehmung an der Innenseite des gegenüberliegenden Schenkels eingreift.

Durch diese Maßnahme wird die definierte Anpressfläche zwischen dem Schenkel der Dichtmembran und dem Schenkel des Gehäusekörpers, zwischen den die Wandung der Rohrprothese geklemmt ist, vergrößert, was die Dichtwirkung insgesamt erhöhen kann.

Für spezielle Anwendungsfälle ist zusätzlich eine dritte Variante der Abdichtvorrichtung denkbar. Hierbei ist vorgesehen, dass die Abdichtvorrichtung einen ringförmigen Gehäusekörper mit rechteckigem Querschnitt aufweist, bei der an der nach oben gerichteten Schmalseite des Querschnittes eine Ausnehmung zur Aufnahme eines Vorsprunges des Gehäuses (Außenformteil) angeordnet ist und bei der an der nach außen gerichteten Längsseite des Querschnittes eine mit einem druckempfindlichen Material gefüllte Nut ausgespart ist.

Diese Gestaltung ermöglicht es, die Abdichtvorrichtung direkt in das Ende einer Aorta einzuführen, so dass diese den Gehäusekörper der Abdichtvorrichtung zumindest im Bereich der mit elastischen Material gefüllten Nut außen überdeckt. Außen an der Aortenwandung wird anschließend ein Federelement beispielsweise in Form eines Federringes platziert, der die Aortenwandung an dem druckempfindlichen Bereich der Abdichtvorrichtung zur Anlage bringt und gegebenenfalls ein leichtes Eindrücken des entsprechenden Bereiches bewirkt. Diese Gestaltung der Abdichtvorrichtung ermöglicht es, eine Prüfung von Aortenklappen gegebenenfalls vor Durchführung einer entsprechenden Operation vornehmen zu können und erweitert somit das Einsatzgebiet der erfindungsgemäßen Vorrichtung nicht unwesentlich.

### Figurenbeschreibung

Nachfolgend werden Ausführungsbeispiele der Ausgestaltung der Erfindung anhand der beigefügten Zeichnungen näher erläutert. Es zeigt:
- Figur 1: einen Querschnitt durch ein erstes Ausführungsbeispiel der erfindungsgemäßen Vorrichtung,
- Figur 2: einen Querschnitt durch die Ausgestaltungsvariante der Figur 1 im Bereich der Schnittlinie AA,
- Figur 3: einen Querschnitt durch die zur erfindungsgemäßen Vorrichtung gehörende Abdichtvorrichtung in einer alternativen Variante zu derjenigen der Figur 1 und
- Figur 4: eine weitere Ausgestaltungsvariante der Abdichtvorrichtung zum Einsatz direkt in Verbindung mit dem Ende einer Aortenblutbahn

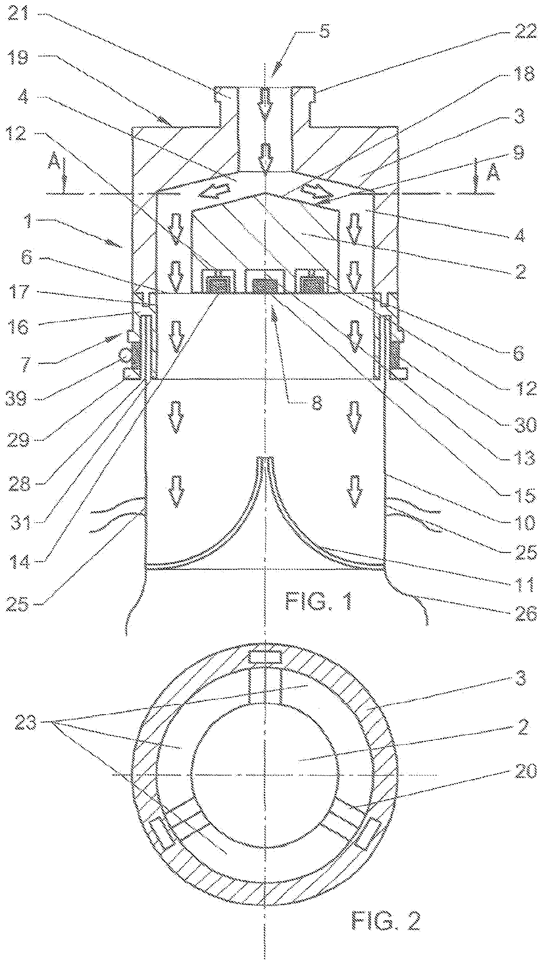

Die in der Figur 1 dargestellte erste Ausführungsvariante der erfindungsgemäßen Vorrichtung zur Prüfung der Funktion einer Aortenklappe 11 besitzt als wesentliche Elemente ein insgesamt mit 1 bezeichnetes Gehäuse und eine insgesamt mit 7 bezeichnete Abdichtvorrichtung.

Die erfindungsgemäße Vorrichtung wird zur Prüfung der Funktion der Aortenklappe 11 entsprechend einer ersten Anwendung auf das freie Ende einer Rohrprothese 10 aufgesetzt. Die Rohrprothese 10 besteht aus Kunststoff und ist an ihrem dem oberen freien Ende abgewandten unteren Ende mit dem Ende einer aufgetrennten Aorta eines Patienten vernäht. In diesem unteren Bereich befinden sich zusätzlich Aderverbindungen 25 zum Herzkreislauf des Patienten, die ebenfalls während der Gesamtoperation mit der Rohrprothese 10 vernäht werden. Sowohl die Aderverbindungen 25 als auch die Verbindung zwischen Rohrprothese 10 und der Aortenwandung 26 müssen absolut flüssigkeitsdicht ausgeführt werden.

Die erfindungsgemäße Vorrichtung dient zum einen zur Überprüfung der Anbindung der Rohrprothese an das körpereigene Gewebe des Patienten und zum anderen zur Überprüfung der Funktion der im unteren Bereich der Rohrprothese 10 angeordneten Aortenklappe 11.

Das Gehäuse 1 der erfindungsgemäßen Vorrichtung besitzt im wesentlichen eine zylinderförmige Außengestalt und besteht aus einem Innenformteil 2 sowie einem das Innenformteil 2 umgebenden glockenförmigen Außenformteil 3. Das Innenformteil 2 besitzt an seiner der Aortenklappe 11 und der Rohrprothese 10 zugewandten Unterseite 8 ein zentralen Druckmesssensor 13 sowie hierzu benachbart im vorliegenden Ausführungsbeispiel zwei optische Sensorelemente 12. Die Aufnahmeöffnung 15 des Druckmesssensors 13 sowie die Messflächen 14 der optischen Sensorelemente 12 liegen dabei zur Unterseite 8 des Innenformteiles 2 offen.

Aus Gründen der Übersichtlichkeit wurde in der Fig. 1 auf die nähere Andeutung der Verkabelung zur Übertragung der Messwerte der optischen Sensorelemente 12 und des Druckmesssensors 13 verzichtet. Eine vorteilhafte Übertragungsart der Messwerte stellt die heute für den Fachmann problemlos realisierbare kabellose Übertragung beispielsweise via Bluetooth dar.

An der den optischen Sensorelementen 12 und dem Druckmesssensor 13 abgewandten Oberseite besitzt das Innenformteil 2 eine kegelförmige mittig zugespitzte Diffusorfläche 18. Die Diffusorfläche 18 ist Bestandteil eines Durchflusskanals 4, der zwischen der Außenseite des Innenformteiles 2 und der Innenseite des glockenförmigen Außenformteiles 3 gebildet ist. Das Außenformteil 3 besitzt an der der Aortenklappe 11 abgewandten Oberseite eine Eingangsöffnung 5. Die Eingangsöffnung 5 ist mit einem Anschlussstutzen 21 versehen und besitzt einen umlaufenden Kragen 22, auf den eine in der Abbildung nicht näher dargestellte Versorgungseinheit für Kontrollflüssigkeit mittels eines Anschlussteiles angeschlossen werden kann.

Der Durchflusskanal 4 verzweigt sich unterhalb der Eingangsöffnung 5, wobei die Diffusorfläche 18 des Innenformteiles 2 den Kontrollflüssigkeitsstrom, der durch die in der Figur 1 eingezeichneten Pfeile verdeutlicht ist, in den ringförmigen Durchflusskanal 4 verteilt.

Wie sich aus der Schnittzeichnung der Figur 2 ergibt, ist das Innenformteil 2 mit dem Außenformteil 3 durch drei über den Umfang gleichmäßig verteilte Stege 20 verbunden. Durch diese konstruktive Gestaltung ergeben sich über den Umfang des Durchflusskanals verteilt drei Ringkanalabschnitte 23.

Zur Aortenklappe 11 hin öffnet sich der Durchflusskanal 4 in Form der Auslassöffnung 6, durch die Kontrollflüssigkeit aus der erfindungsgemäßen Vorrichtung in den Raum unterhalb des Innenformteiles 2 strömen kann. der sich im Inneren der Rohrprothese 10 ergibt.

Der Anschluss zwischen dem Gehäuse 1 und der Oberkante der Rohrprothese 10 wird mittels der Abdichtvorrichtung 7 vorgenommen. Die Abdichtvorrichtung 7 dient dabei durch ihren speziellen Aufbau dazu, eine flüssigkeitsdichte Abschottung des Innenraums der Rohrprothese 10 gegenüber der Umgebung sicherzustellen.

Für unterschiedliche Anwendungen sind verschiedene Aufbaumöglichkeiten und konstruktive Ausbildungen der Abdichtvorrichtung 7 in den Figuren 1, 3 und 4 dargestellt.

Die Abdichtvorrichtung 7, wie sie in der Figur 1 gezeigt ist, besteht im wesentlichen aus einem ringförmig ausgebildeten Gehäusekörper 16, der im wesentlichen einen rechteckigen Querschnitt aufweist und an das Außenformteil 3 mittels einer nicht näher dargestellten und aus dem Stand der Technik hinlänglich bekannten Fixiervorrichtung beispielsweise durch eine Verrastung angebunden ist.

Wie aus der Figur 1 zu entnehmen ist, ist an der oberen, dem Außenformteil 3 zugewandten Schmalseite des rechteckigen Querschnittes des Gehäusekörpers 16 eine U-förmige Ausnehmung 24 angeordnet, die umlaufend ausgestaltet sein kann oder nur in einzelnen Kreissegmenten des Gehäusekörpers 16 vorhanden sein kann. In die Ausnehmung 24 greift ein Vorsprung 27 an der Unterseite des Außenformteiles 3 ein. Diese konstruktive Gestaltung unterstützt die Steifigkeit und Dichtigkeit der Verbindung zwischen Außenformteil 3 und Gehäusekörper 16.

An der gegenüberliegenden Schmalseite des Gehäusekörpers befindet sich über den Umfang umlaufend ebenfalls eine U-förmige Ausnehmung 28. In dieser Ausnehmung 28 greift der obere Rand der Rohrprothese 10 ein, so dass er auf der Innenseite an der Innenseite des einen Schenkels 31 der U-förmigen Ausnehmung 24 zur Anlage kommt und auf der gegenüberliegenden Außenseite vom anderen Schenkel 29 der U-förmigen Ausnehmung 28.

Um eine entsprechende Dichtigkeit zwischen oberem Rand der Rohrprothese 10 und der Abdichtvorrichtung 7 bereitzustellen, ist dieser äußere Schenkel 29 zumindest in einem Teilbereich 30 aus einem elastischen Material gefertigt. Konstruktiv ist dieser Teilbereich 30 so konzipiert, dass das Material in Richtung des parallel beabstandeten anderen Schenkels 29 der Ausnehmung 28 vorgespannt ist, so dass der obere Rand der Rohrprothese 10 zwischen den beiden Schenkeln 29 und 31 eingespannt wird, sobald die Abdichtvorrichtung 7 auf den oberen Rand der Rohrprothese 10 aufgesetzt ist. Die Anpresskräfte im Teilbereich 30 können zusätzlich dadurch unterstützt werden, dass an der Außenseite des Teilbereiches 30 ein Federelement in Form eines umlaufenden Federringes 39 aufgesetzt wird.

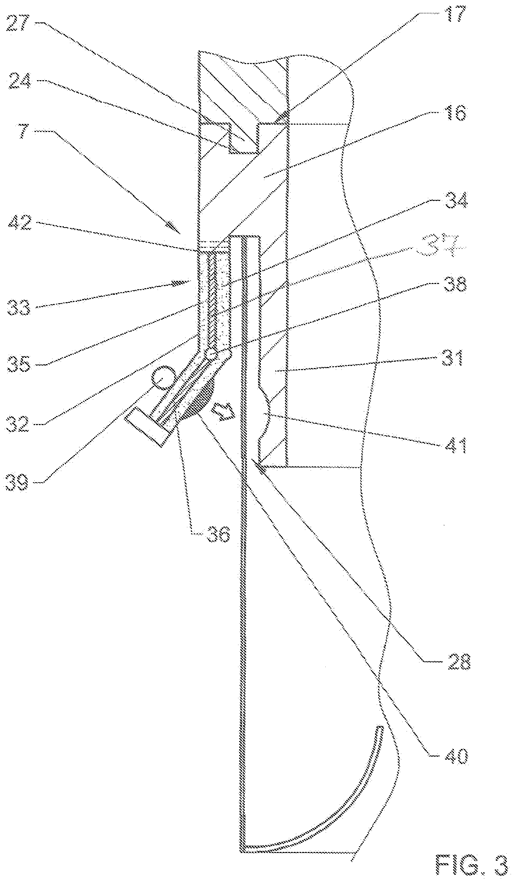

In der Darstellung der Figur 3 ist in vergrößertem Maßstab eine weitere Ausgestaltungsvariante der Abdichtvorrichtung 7 dargestellt. Die Abdichtvorrichtung 7 besteht dabei ebenso wie in der Variante aus Figur 1 aus einem umlaufenden Gehäusekörper 16, der an seiner oberen Schmalseite mit einer Ausnehmung 24 versehen ist, in der ein Vorsprung 27 als Teil des Außenformteiles 3 eingreift. An der der Schmalseite 17 gegenüberliegenden Schmalseite befindet sich analog zur Variante der Figur 1 eine U-förmige Ausnehmung 28 mit einem innenliegenden Schenkel 31 und einem außenliegenden Schenkel 32.

Im Unterschied zur Variante 1 ist die am Außenschenkel 32 befindliche Dichtmembran 33 doppelwandig gestaltet und definiert zwischen ihren parallel beabstandeten Wänden 34 und 35 einen Zwischenraum 36. In diesem Zwischenraum 36, der über den gesamten Umfang des äußeren Schenkels 32 verläuft, sind eine Mehrzahl von Federstäben 37 angeordnet. Diese Federstäbe 37 bewirken im aufgesetzten Zustand der Abdichtvorrichtung 7 auf den Außenrand der Rohrprothese 10 einen Andruck an die Außenseite der Rohrprothese 10.

Aus der Figur 3 ist im Hinblick auf die Gestaltung der Federstäbe 37 die Besonderheit zu ersehen, dass diese Federstäbe 37 etwa in der Mitte ihrer Längserstreckung ein Knickgelenk 38 aufweisen. Dieses Knickgelenk 38 bewirkt die Möglichkeit einer Winkellage zwischen dem im wesentlichen parallel zur Außenwandung der Rohrprothese 10 verlaufenden oberen Teil der Dichtmembran 33 und dem unterhalb des Knickgelenkes 38 gelegenen zweiten Bereich der Dichtmembran 33. Durch diese Gestaltung ist die Möglichkeit gegeben, dass der Abstand zwischen den Schenkeln 31 und 32 der U-förmigen Ausnehmung 28 zur Öffnung hin breiter ist als im oberen Bereich. Diese Maßnahme erleichtert das Aufsetzen der Abdichtvorrichtung 7 auf das obere freie Ende der Rohrprothese 10. Ist das Aufsetzen erfolgreich abgeschlossen, wird bedingt durch das Knickgelenk 38 der untere Teil der Dichtmembran 33 an die Außenseite des oberen Randes der Rohrprothese 10 herangeklappt. Dies kann beispielsweise unter Zuhilfenahme eines Federringes 39 geschehen, wie er bereits in der Darstellung der Figur 1 abgebildet ist.

Die Dichtwirkung der Abdichtvorrichtung 7 kann noch zusätzlich dadurch gesteigert werden, dass an der Innenseite der Dichtmembran 33 ein vorstehender Druckwulst 40 angeordnet wird, der nach Anklappen des unteren Teiles der Dichtmembran 33 an die Außenseite der Rohrprothese 10 zur Anlage kommt und diese in eine korrespondierende Ausnehmung 41 an der Innenseite des gegenüberliegenden Schenkels 31 drückt.

Um den Flüssigkeitsdruck innerhalb der Ausnehmung 28 auszugleichen, befindet sich im Bodenbereich dieser Ausnehmung 28 eine oder mehrere Bohrungen 42.

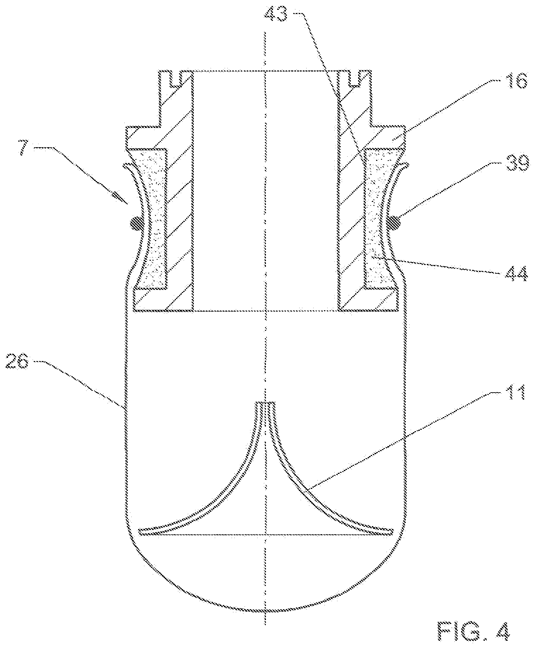

Eine dritte Variante der Abdichtvorrichtung 7 ist in der Figur 4 dargestellt. In diesem Ausführungsbeispiel ist an der der Schmalseite 17 gegenüberliegenden Unterseite keine U-förmige Ausnehmung vorhanden. Stattdessen befindet sich an der äußeren Breitseite des Gehäusekörpers 16 der Abdichtvorrichtung 7 eine Nut 43, die mit einem Formkörper 44 aus elastischem Material gefüllt ist.

Diese Variante der Abdichtvorrichtung 7 wird dann eingesetzt, wenn eine Überprüfung einer Aortenklappe vorgenommen werden soll und keine Rohrprothese 10 wie in den vorher beschriebenen Ausführungsbeispielen vorhanden ist.

Die Aortenwandung 26 wird bei diesem Ausführungsbeispiel über das freie untere Ende des Gehäusekörpers 16 herübergestülpt, so dass sie im Bereich der Nut 43 an den darin angeordneten Formkörper 44 zur Anlage kommt. Ist dies geschehen, so wird wie in den anderen Ausführungsbeispielen oben beschrieben, ein Federring 39 an der Außenseite der Aortenwandung 26 zur Anlage gebracht, der die Aortenwandung 26 gegen den Formkörper 44 drückt.

Wie aus der Figur 4 schematisch dargestellt, drückt der Federring 39 die Aortenwandung 26 aufgrund der elastischen Eigenschaften des Formkörpers 24 in diesen ein, so dass eine Abdichtung des oberen Endes der Aortenwandung 26 mit der erfindungsgemäßen Vorrichtung herbeigeführt wird. Anschließend kann über das an der Abdichtvorrichtung 7 mittels der Fixiervorrichtung festgelegten Gehäuse der Bereich zwischen Aortenklappe 11 und Unterseite des Innenformteiles 2 des Gehäuses 1 befindliche Zwischenraum mit Kontrollflüssigkeit beaufschlagt werden, so dass durch die Druckmesssensoren 12 und das optische Sensorelement 13 die Funktion der Aortenklappe 11 visuell und messtechnisch überprüft werden kann.

### Bezugszeichenliste:

- 1: Gehäuse
- 2: Innenformteil
- 3: Außenformteil
- 4: Durchflusskanal
- 5: Eingangsöffnung
- 6: Auslassöffnung
- 7: Abdichtvorrichtung
- **8**: Unterseite
- 9: Oberseite
- 10: Rohrprothese
- 11: Aortenklappe
- 12: optisches Sensorelement
- 13: Druckmesssensor
- 14: Aufnahmeöffnung (optisches Sensorelement)
- 15: Messfläche (Druckmesssensor)
- 16: Gehäusekörper
- 17: Schmalseite
- 18: Diffusorfläche
- 19: Oberseite
- 20: Steg
- 21: Anschlussstutzen
- 22: Kragen
- 23: Ringkanalabschnitt
- 24: Ausnehmung
- 25: Aderanschluß
- 26: Aortenwandung
- 27: Vorsprung
- 28: Ausnehmung
- 29: Äußerer Schenkel
- 30: Teilbereich
- 31: Schenkel
- 32: Schenkel
- 33: Dichtmembran
- 34: Wand
- 35: Wand
- 36: Zwischenraum
- 37: Federstab
- 38: Knickgelenk
- 39: Federung
- 40: Druckwulst
- 41: Ausnehmung
- 42: Bohrung
- 43: Nut
- 44: Formkörper

## Patentansprüche

1. Vorrichtung zur Prüfung der Funktion einer Aortenklappe durch Aufsetzen der Vorrichtung auf das freie Ende einer zylinderförmigen in die Aorta eingesetzten Rohrprothese, welche mit ihrem anderen Ende an die Aortenwand im Bereich der Aortenklappe angebunden ist,
**dadurch gekennzeichnet, dass**
die Vorrichtung aus einem mehrteiligen, mit mindestens einem Durchflusskanal (4) für Kontrollflüssigkeit versehenem Gehäuse (1) besteht, wobei das Gehäuse (1) an seiner dem freien Ende der Rohrprothese (10) zugewandten Unterseite (8) mit einer umlaufenden, auf den Rand der Rohrprothese (10) aufsetzbaren Abdichtvorrichtung (7) versehen ist und wobei an der Unterseite (8) mindestens ein Druckmesssensor (13) und mindestens ein optisches Sensorelement (12) zur Videoaufnahme angeordnet ist.

2. Vorrichtung nach Anspruch 1,
**dadurch gekennzeichnet, dass**
der Durchflusskanal (4) ein an der dem freien Ende der Rohrprothese (10) abgewandten Oberseite (9) des Gehäuses (1) angeordnete Eingangsöffnung (5) aufweist und sich innerhalb des Gehäuses (1) zu einem Ringkanal verzweigt, der an der Unterseite des Gehäuses (1) innerhalb der umlaufenden Abdichtvorrichtung (7) in eine ringförmige Auslassöffnung (6) mündet.

3. Vorrichtung nach Anspruch 2,
**dadurch gekennzeichnet, dass**
die Innenseite des Ringkanals durch ein zentrales Innenformteil (2) als Bestandteil des Gehäuses (1) gebildet ist, in das der Druckmesssensor (13) und das optische Sensorelement (12) aufgenommen sind, wobei die Messfläche (15) des Druckmesssensors (13) und die Aufnahmeöffnung (14) des optischen Sensorelementes (12) an der Unterseite offenliegen.

4. Vorrichtung nach einem der Ansprüche 2 oder 3,
**dadurch gekennzeichnet, dass**
die Außenseite des Durchflusskanals (4) durch ein das zentrale Innenformteil (2) beanstandet umgebendes glockenförmiges Außenformteil (3) gebildet ist, in der die Eingangsöffnung (5) des Durchflusskanals (4) angeordnet ist.

5. Vorrichtung nach Anspruch 4,
**dadurch gekennzeichnet, dass**
die Abdichtvorrichtung (7) am Außenformteil (3) abnehmbar durch eine Fixiervorrichtung festgelegt ist.

6. Vorrichtung nach einem der Ansprüche 1 bis 5,
**dadurch gekennzeichnet, dass**
die Abdichtvorrichtung (7) einen ringförmigen Gehäusekörper (16) aufweist, der einen rechteckförmigen Querschnitt besitzt und bei dem an einem der gegenüberliegenden Schmalseiten (17) des Querschnittes eine Ausnehmung (24) zur Aufnahme eines Vorsprunges (27) des Außenformteiles (3) angeordnet ist und bei der sich an der gegenüberliegenden Schmalseite eine U-förmige Ausnehmung (28) zur Aufnahme des ringförmigen oberen Endes der Rohrprothese (10) befindet.

7. Vorrichtung nach Anspruch 6,
**dadurch gekennzeichnet, dass**
der äußere Schenkel (29) der U-förmigen Ausnehmung (28) zur Aufnahme der Rohrprothese (10) mindestens eine elastische Dichtmembran (33) aufweist, die in Richtung des gegenüberliegenden Schenkels (31) der U-förmigen Ausnehmung (28) vorgespannt ist.

8. Vorrichtung nach Anspruch 7,
**dadurch gekennzeichnet, dass**
die elastische Dichtmembran (33) doppelwandig ausgeführt ist und sich zwischen den parallel beabstandeten Wänden (34, 35) ein Zwischenraum (36) befindet, in den eine Mehrzahl von Federstäben (37) über den Umfang des umlaufenden Zwischenraumes (36) verteilt angeordnet sind, die die Vorspannung der Dichtmembran (33) bewirken.

9. Vorrichtung nach Anspruch 8,
**dadurch gekennzeichnet, dass**
die Federstäbe (37) jeweils ein Knickgelenk (38) aufweisen, die eine Winkellage des Teiles der Dichtmembran (33) in Richtung des Gehäuses (1) oberhalb des Knickgelenkes (38) zu demjenigen Teil der Dichtmembran (33) unterhalb des Knickgelenkes (38) ermöglichen.

10. Vorrichtung nach einem der Ansprüche 7 bis 9,
**dadurch gekennzeichnet, dass**
die zum gegenüberliegenden Schenkel (31) der U-förmigen Ausnehmung (28) zur Aufnahme der Rohrprothese (10) weisende Innenfläche der umlaufenden Dichtmembran (33) einen über die Innenfläche vorstehende umlaufenden Druckwulst (40) aufweist, der in aufgesetztem Zustand der Vorrichtung auf das freie Ende der Rohrprothese (10) in eine korrespondierende Ausnehmung (41) an der Innenseite des gegenüberliegenden Schenkels (31) eingreift.

11. Vorrichtung nach einem der Ansprüche 1 bis 10,
**dadurch gekennzeichnet, dass**
das Innenformteil (2) aus Metall, vorzugsweise Edelstahl hergestellt ist.

12. Vorrichtung nach einem der Ansprüche 1 bis 11,
**dadurch gekennzeichnet, dass**
das Außenformteil (3) des Gehäuses (1) und der Gehäusekörper (16) der Abdichtvorrichtung (7) aus Kunststoff bestehen.

13. Vorrichtung nach einem der Ansprüche 1 bis 5,
**dadurch gekennzeichnet, dass**
die Abdichtvorrichtung (7) einen ringförmigen Gehäusekörper (16) mit rechteckigem Querschnitt aufweist, bei dem an einem der Schmalseiten des Querschnittes eine Ausnehmung (24) zur Aufnahme eines Vorsprungs (27) des Außenformteiles (3) angeordnet ist und bei dem an der nach außen gerichteten Längsseite des Querschnittes eine mit einem druckempfindlichen Formkörper (44) gefüllte Nut (43) ausgespart ist.

## Claims

1. A device for checking the function of an aortic valve by mounting the device on the free end of a cylindrical tubular prosthesis inserted into the aorta, the other end of which is connected to the aortic wall in the region of the aortic valve,
**characterized in that**
the device consists of a multipart housing (1) provided with at least one flow channel (4) for inspection fluid, wherein the housing (1) is provided on its underside (8) facing the free end of the tubular prosthesis (10) with a peripheral sealing device (7) that can be mounted on the edge of the tubular prosthesis (10), and wherein at least one pressure measuring sensor (13) and at least one optical sensor element (12) are provided on the underside (8) for recording video.

2. The device according to claim 1,
**characterized in that**
the flow channel (4) has an inlet opening (5) arranged in the top side (9) of the housing (1) facing away from the free end of the tubular prosthesis (10) and branches within the housing (1) to a ring channel that terminates in an annular outlet opening (6) in the underside of the housing (1) within the peripheral sealing device (7).

3. The device according to claim 2,
**characterized in that**
the inside of the ring channel is formed by a central inner molded part (2) as a component of the housing (1) in which the pressure measuring sensor (13) and the optical sensor element (12) are accommodated, wherein the measuring surface (15) of the pressure measuring sensor (13) and the seating opening (14) of the optical sensor element (12) in the underside are exposed.

4. The device according to one of claims 2 or 3,
**characterized in that**
the outside of the flow channel (4) is formed by a bell-shaped outer molded part (3) that surrounds the central inner molded part (2) at a distance and in which the inlet opening (5) of the ring channel (4) is arranged.

5. The device according to claim 4,
**characterized in that**
the sealing device (7) is removably affixed on the outer molded part (3) by means of a fixing device.

6. The device according to one of claims 1 to 5,
**characterized in that**
the sealing device (7) has an annular housing body (16) which possesses a rectangular cross-section, and in which a recess (24) is arranged for accommodating a projection (27) of the outer molded part (3) in one of the opposing narrow sides (17) of the cross-section, and in which a U-shaped recess (28) is located in the opposing narrow side for accommodating the annular upper end of the tubular prosthesis (10).

7. The device according to claim 6,
**characterized in that**
the outer leg (29) of the U-shaped recess (28) has at least one elastic sealing membrane (33) for accommodating the tubular prosthesis (10) and which is pretensioned in the direction of the opposing leg (31) of the U-shaped recess (28).

8. The device according to claim 7,
**characterized in that**
the elastic sealing membrane (33) is designed double-walled, and a gap (36) is located between the parallel spaced walls (34, 35) in which a plurality of spring rods (37) are arranged distributed over the perimeter of the peripheral gap (36) which cause the pretension of the sealing membrane (33).

9. The device according to claim 8,
**characterized in that**
the spring rods (37) each have a swivel joint (38) that enables an angled position of the part of the sealing membrane (33) toward the housing (1) above the swivel joint (38) relative to the part of the sealing membrane (33) below the swivel joint (38).

10. The device according to one of claims 7 to 9,
**characterized in that**
the inner surface of the peripheral sealing membrane (33) facing the opposing leg (31) of the U-shaped recess (28) for accommodating the tubular prosthesis (10) has a peripheral press bead (40) projecting above the inner surface that, when the device is in a mounted state on the free end of the tubular prosthesis (10), engages in a corresponding recess (41) in the inner side of the opposing leg (31).

11. The device according to one of claims 1 to 10,
**characterized in that**
the inner molded part (2) is made of metal, preferably high-grade steel.

12. The device according to one of claims 1 to 11,
**characterized in that**
the outer molded part (3) of the housing (1) and the housing body (16) of the sealing device (7) consist of plastic.

13. The device according to one of claims 1 to 5,
**characterized in that**
the sealing device (7) has an annular housing body (16) with a rectangular cross-section in which a recess (24) is arranged in one of the narrow sides of the cross-section for accommodating a projection (27) of the outer molded part (3), and in which a groove (43) filled with a pressure-sensitive molded body (44) is cut out in the outwardly directed longitudinal side of the cross-section.

## Revendications

1. Dispositif pour le contrôle du fonctionnement d'une valve aortique en posant ce dispositif sur l'extrémité libre d'une prothèse tubulaire cylindrique dans l'aorte, laquelle est reliée avec son autre extrémité à la paroi de l'aorte dans la région de la valve aortique,
**caractérisé en ce que**
ce dispositif est constitué d'un boîtier (1) doté d'au moins un canal d'écoulement (4) pour le liquide de contrôle, le boîtier (1) étant doté sur sa face inférieure (8) orientée vers l'extrémité libre de la prothèse tubulaire (10) d'un dispositif d'étanchéité (7) périphérique pouvant être posé sur le bord de la prothèse tubulaire (10) et sur la face inférieure (8) étant agencés au moins un capteur de mesure de pression (13) et au moins un élément de capteur optique (12) pour l'enregistrement vidéo.

2. Dispositif selon la revendication 1,
**caractérisé en ce que**
le canal d'écoulement (4) présente une ouverture d'entrée (5) disposée sur la face supérieure (9) du boîtier (1) opposée à l'extrémité libre de la prothèse tubulaire (10) et, à l'intérieur du boîtier (1), se ramifie pour former un canal annulaire qui débouche sur la face inférieure du boîtier (1) à l'intérieur du dispositif d'étanchéité périphérique (7) dans une ouverture d'évacuation annulaire (6).

3. Dispositif selon la revendication 2,
**caractérisé en ce que**
la face interne du canal annulaire est formée par une pièce moulée intérieure centrale (2) faisant partie du boîtier (1) dans laquelle le capteur de mesure de pression (13) et l'élément de capteur optique (12) sont logés, la face de mesure (15) du capteur de mesure de pression (13) et l'élément de capteur optique (12) étant exposés au niveau de la face inférieure.

4. Dispositif selon une des revendications 2 ou 3,
**caractérisé en ce que**
la face externe du canal d'écoulement (4) est formée par une pièce moulée extérieure (3) en forme de cloche entourant à distance la pièce moulée intérieure centrale (2), dans laquelle l'ouverture d'entrée (5) du canal d'écoulement (4) est agencée.

5. Dispositif selon la revendication 4,
**caractérisé en ce que**
le dispositif d'étanchéité (7) est fixé de façon amovible sur la pièce moulée extérieure (3) par un dispositif de fixation.

6. Dispositif selon une des revendications 1 à 5,
**caractérisé en ce que**
le dispositif d'étanchéité (7) présente un corps de boîtier annulaire (16) qui possède une section rectangulaire et dans lequel, sur une des faces étroites (17) opposées de la section, un évidement (24) est disposé pour recevoir une protubérance (27) de la pièce moulée extérieure (3) et, sur la face étroite opposée, un évidement en forme de U (28) se trouve pour recevoir l'extrémité supérieure annulaire de la prothèse tubulaire (10).

7. Dispositif selon la revendication 6,
**caractérisé en ce que**
la patte extérieure (29) de l'évidement en forme de U (28) pour recevoir la prothèse tubulaire (10) présente au moins une membrane d'étanchéité élastique (33) qui est précontrainte en direction de la patte opposée (31) de l'évidement en forme de U (28).

8. Dispositif selon la revendication 7,
**caractérisé en ce que**
la membrane d'étanchéité élastique (33) est à double paroi et qu'entre les parois parallèles espacées (34, 35) se trouve un interstice (36) dans lequel une multitude de barrettes élastiques (37) sont disposées sur la périphérie de l'interstice périphérique (36) et exercent la précontrainte de la membrane d'étanchéité (33).

9. Dispositif selon la revendication 8,
**caractérisé en ce que**
les barrettes élastiques (37) présentent chacune une articulation (38), lesquelles permettent une position angulaire de la partie de la membrane d'étanchéité (33) située au-dessus de l'articulation (38) en direction du boîtier (1) par rapport à la partie de la membrane d'étanchéité (33) située au-dessous de l'articulation (38).

10. Dispositif selon une des revendications 7 à 9,
**caractérisé en ce que**
la face interne de la membrane d'étanchéité périphérique (33) tournée vers la patte opposée (31) de l'évidement en forme de U (28) pour recevoir la prothèse tubulaire (10) présente un bourrelet de pression périphérique (40) faisant saillie par-dessus la face interne qui, lorsque le dispositif est posé sur l'extrémité libre de la prothèse tubulaire (10), pénètre dans un évidement correspondant (41) sur la face interne de la patte opposée (31).

11. Dispositif selon une des revendications 1 à 10,
**caractérisé en ce que**
la pièce moulée intérieure (2) est fabriquée en métal, de préférence en acier inoxydable.

12. Dispositif selon une des revendications 1 à 11,
**caractérisé en ce que**
la pièce moulée extérieure (3) du boîtier (1) et le corps de boîtier (16) du dispositif d'étanchéité (7) sont en matière plastique.

13. Dispositif selon une des revendications 1 à 5,
**caractérisé en ce que**
le dispositif d'étanchéité (7) présente un corps de boîtier annulaire (16) de section rectangulaire dans lequel, sur une des faces étroites de la section, un évidement (24) est disposé pour recevoir une protubérance (27) de la pièce moulée extérieure (3) et dans lequel, sur la face longitudinal de la section orientée vers l'extérieur, une rainure (43) remplie avec un corps moulé sensible à la pression (44) est ménagée.
